# EUROPEAN PATENT APPLICATION

(11) **EP 1 600 107 A1**
(43) Date of publication of application: **30.11.2005**
(21) Application number: 04712682.6
(22) Date of filing: 19.02.2004
(51) Int. Cl.: A61B 17/00

(54) **WIRE DEVICE FOR MEDICAL USE**

(30) Priority: 20.02.2003 JP 2003042726
(71) Applicant: Kaneka Medix Corporation, Osaka-shi, Osaka 530-8288 (JP)
(72) Inventor: Sakai, Shinichi, c/o Kaneka Medix Corporation, Kanagawa 258-0113 (JP); Ogawa, Atsushi, c/o Kaneka Medix Corporation, Kanagawa 258-0113 (JP)
(74) Representative: Grünecker, Kinkeldey, Stockmair & Schwanhäusser Anwaltssozietät
(86) International application number: PCT/JP2004/001855
(87) International publication number: WO 2004/073525

(57) **Abstract**

Disclosed herein is a novel medical wire device which is equipped with a filter unit, capable of surely capturing free embolismic debris upon angioplasty of a constriction lesion in a lumen, capable of easily confirming a developed state of a filter under radiography by a surgeon, and thus improved in function of the filter unit.

The medical wire device of the present invention comprises a filter unit which is to be located, upon angioplasty of a constriction lesion in a lumen, on a distal side from the constriction lesion for capturing free embolismic debris separated from an inner wall of the constriction lesion, and a wire provided with the filter unit at its distal-side end. A filter is made up of a metal having radiopaque property.

## Description

### TECHNICAL FIELD

The present invention relates to a medical wire device used in, for example, angioplasty for a constriction lesion in a blood vessel and other lumens in a vital body.

### BACKGROUND ART

As a treatment method for dilating or reopening a constriction lesion in a blood vessel or any other lumen, which has been formed by deposition of embolismic debris such as plaque or other substances on a vessel wall, there is currently known angioplasty. In such angioplasty, for example, a method of forming a lumen by introducing an expansible balloon into a constriction lesion in a contracted state and expanding the balloon, a method of forming a lumen by introducing a stent in a contracted state in a diameter into a constriction lesion and expanding it, or the like is utilized.

In such angioplasty, in some cases, embolismic debris located at a constriction lesion in a lumen may unexpectedly separate from an inner wall of the lumen and be caused to flow in a peripheral region upon, for example, the expansion of the balloon or the expansion of the stent. As a result, there is a possibility that the free embolismic debris may be deposited on another site in the lumen to form a thrombus at the site or may reach a vital organ to adversely affect it.

In order to solve such a problem, it is conducted to arrange a medical implement for capturing the free embolismic debris in a distal-side region from the constriction lesion when the angioplasty is performed. There is known, for example, an intravascular filter device comprising a filter for capturing free embolismic debris, which are likely to occur during angioplasty, provided at a distal end of a guide wire, and used by introducing the filter into a lumen in a folded state and developing it at an intended site on a distal side from the constriction lesion by a proper developing means (see Prior Art. 1).

The filter making up this intravascular filter device is composed of, for example, a polymer or a physiologically acceptable metal or alloy, has a size not to impede a blood stream when the filter is developed, and is formed in mesh having a small opening diameter so as to permit surely capturing free embolismic debris.

In general, a filter device used in, for example, a lumen such as a blood vessel is required to have, for example, the following properties (1) to (3):
(1) It is to have a means for confirming that a filter has been surely developed at a desired site in a blood vessel in a state brought into close contact with an inner wall surface without any clearance.
(2) Since it may be necessary in some cases to introduce a filter into, for example, a blood vessel having a diameter as small as at most 3 mm and a complicated form, the whole of a filter unit is to have high flexibility.
(3) Since embolismic debris have a possibility of forming a thrombus in a blood vessel or the like in, for example, a cerebral region even when they are minute, the filter is to have a small opening diameter in order to surely capture embolismic debris including such minute ones.

Prior Art. 1: Japanese Patent Application (KOHYO) No. 2000-504263 (through PCT route)

### DISCLOSURE OF THE INVENTION

The present invention has been made on the basis of the foregoing circumstances and has as its object the provision of a novel medical wire device which is equipped with a filter unit for capturing free embolismic debris, capable of surely capturing the free embolismic debris upon angioplasty of a constriction lesion in a lumen, capable of easily confirming a developed state of a filter under radiography by a surgeon and thus improved in function of the filter unit.

According to the present invention, there is provided a medical wire device comprising a filter unit which is to be located, upon angioplasty of a constriction lesion in a lumen, on a distal side from the constriction lesion for capturing free embolismic debris separated from an inner wall of the constriction lesion, and a wire provided with the filter unit at its distal-side end,
wherein a filter making up the filter unit is made up of a metal having radiopaque property.

According to the present invention, there is also provided a medical wire device comprising a filter unit which is to be located, upon angioplasty of a constriction lesion in a lumen, on a distal side from the constriction lesion for capturing free embolismic debris separated from an inner wall of the constriction lesion, and a wire provided with the filter unit at its distal-side end,
wherein a filter making up the filter unit is in the form of a mesh composed of a metallic wire material having an element wire diameter of at most 10 µm and has tensile break strength of at least 1,000 N/mm².

The term "free embolismic debris" as used herein means substances caused to flow in a blood stream and having a possibility of forming a thrombus in a lumen, and for example, substances such as plaque, clot and other tissue pieces deposited on an inner wall of a constriction lesion in a lumen.

In the medical wire device according to the present invention, the following embodiments (1) to (9) or combinations of the embodiments (1) to (9) are preferred.
(1) The filter unit comprises a supporting frame formed diametrically contractible and expansible and composed of a metallic wire material, and a filter provided so as to be folded by diametrically contracting the supporting frame and developed by expanding the supporting frame.
(2) The metallic wire material making up the filter is tungsten or a tungsten-containing alloy.
(3) The supporting frame making up the filter unit is formed by providing a plurality of frame components each composed of a metallic wire material so as to extend in a longitudinal direction along a spheroidal surface, and the filter is provided along an inner peripheral surface of a distal-side portion in the supporting frame.
(4) The supporting frame making up the filter unit is in a state that one end of a distal-side end and a proximal-side end has been fixed to the wire, and the other end is movable along a longitudinal direction to the wire, and is so constructed that it becomes a diametrically contracted state by its own bendable property under a state that external force has been applied and becomes an expanded state by being released from the external force.
(5) The supporting frame is made up of a metal having radiopaque property.
(6) The opening diameter of the filter making up the filter unit is 50 to 200 µm.
(7) The opening rate of the filter making up the filter unit is 65 to 99%.
(8) The thickness of the supporting frame is 0.01 to 0.1 mm.
(9) The medical wire device comprises a holding means for holding an intracorporeally indwelling device for formation of a lumen at a position on a proximal side from the filter unit in the wire.

### EFFECTS OF THE INVENTION

According to the medical wire devices of the present invention, the filter making up the filter unit is made up of a metal having radiopaque property, whereby a surgeon is able to easily confirm under radiography that the filter is in a state surely developed in a lumen and brought into close contact with an inner wall surface without any clearance when it is developed.

According to the medical wire devices of the present invention, the filter making up the filter unit is made up of a metal having high strength, whereby the metallic metal material making up the filter can be made diametrally small or shin. As a result, the filter itself can be made thin and made up as that having high flexibility. Accordingly, the filter can be developed or folded following the deformation of the supporting frame, such as expansion or diametrical contraction, without impeding the deformation of the supporting frame, so that the filter can be surely developed to an expected form in a lumen.

In addition, the metallic wire material is made diametrally small or thin, whereby the opening diameter of the filter can be made small while retaining the expected opening rate, so that the embolismic debris can be surely captured under a state that perfusion on a downstream side from a constriction lesion is scarcely impeded. Accordingly, a function as the filter unit can be improved.

In particular, the filter is made up of tungsten or an alloy composed mainly of tungsten, whereby such effects as described above can be surely achieved because tungsten or the alloy composed mainly of tungsten has high radiopaque property upon radiography, high strength and excellent working ability by itself compared with other metals.

In addition, the supporting frame making up the filter unit is made up of the metal having radiopaque property, whereby sufficient effects can be achieved from the viewpoint of practical use.

Further, the supporting frame making up the filter unit is composed of that having a thin wall not thicker than the specified thickness, whereby the function inherent in the filter unit that the free embolismic debris in the lumen are captured can be surely achieved as described above, and, at the same time, the filter unit itself can be made up as that having high flexibility, thereby being capable to deform the filter unit following the form of a lumen into which it should be inserted. As a result, high housing ability into the lumen and high operating ability within the lumen are achieved, and thus the filter unit can be made up as that adequately applicable to, for example, blood vessels having a small inner diameter and a complicated form, or the like.

### BRIEF DESCRIPTION OF THE DRAWINGS

Fig. 1 schematically illustrates the construction of a medical wire device according to an embodiment of the present invention.
Fig. 2 is an explanatory sectional view illustrating a connected state between a core wire and respective frame components.
Fig. 3 schematically illustrates the construction of a medical wire device according to another embodiment of the present invention.

### [Description of characters]

- 10: Medical wire device
- 11: Core wire
- 12A: Proximal-side region
- 12B: Tapered region
- 12C: Distal-side region
- 13A, 13B: Coiled bodies
- 20: Filter unit
- 21: Supporting frame
- 21A: Frame component
- 22: Filter
- 31: Frame component
- 32: Core wire
- 35: Developing means

- 36: Wire body
- 37: Supporting member
- 38: Wire for development

### BEST MODE FOR CARRYING OUT THE INVENTION

The embodiments of the present invention will hereinafter be described in detail with reference to the drawings.

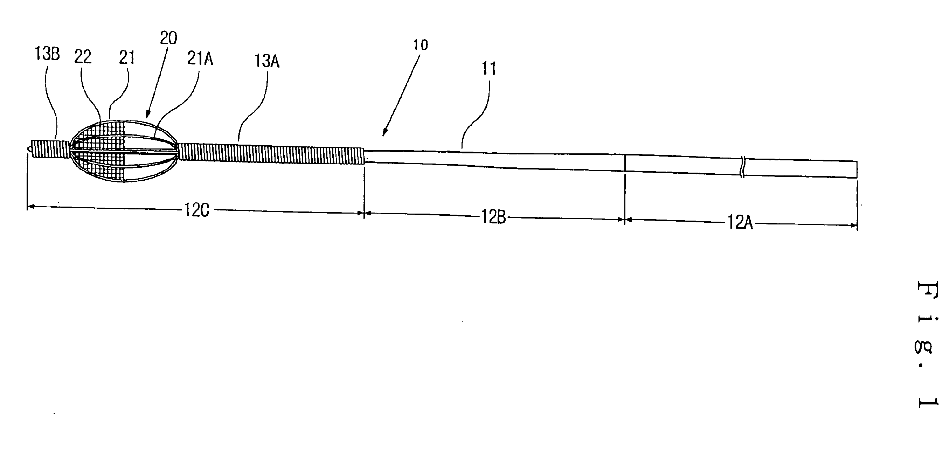

Fig. 1 schematically illustrates the construction of a medical wire device according to an embodiment of the present invention.

The medical wire device 10 basically comprises a filter unit 20, which is to be located, upon, for example, angioplasty of a constriction lesion in a lumen, in a distal- side region from the constriction lesion and equipped with a filter for capturing free embolismic debris separated from an inner wall of the constriction lesion, and a core wire 11 provided with the filter unit 20 at its distal-side end.

The core wire 11 is composed of, for example, stainless steel and comprises a proximal region 12A, a flexible tapered region 12B continuing to the proximal-side region 12A, the outer diameter of which gradually decreases toward the distal side thereof, and a distal-side region 12C continuing to the tapered region 12B, and the filter unit 20 is provided in the distal region 12C.

The filter unit 20 is made up of a supporting frame 21 having a spheroid shape as a whole in a form along the outer peripheral surface of an imaginary spheroid extending in a longitudinal direction, and having a cage-shaped frame structure that is expansible and contractible in a radial direction, and a filter 22 provided on an inner peripheral surface of this supporting frame 21.

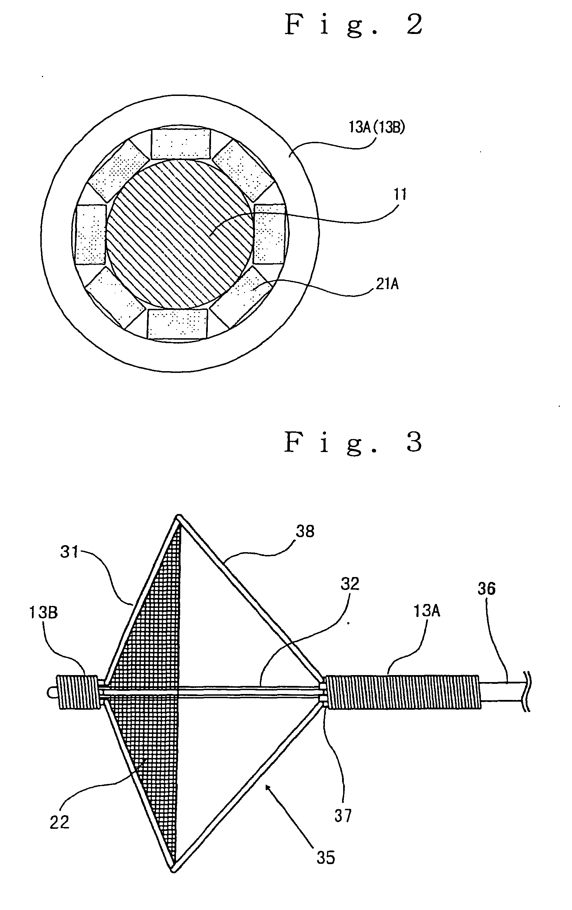

The supporting frame 21 making up the filter unit 20 is formed by arranging a plurality of frame components (8 components in the illustrated embodiment) 21A each composed of, for example, a belt-like metallic wire material so as to extend along a longitudinal direction in a state aligned at equal intervals in a circumferential direction of the spheroid. As illustrated in Fig. 2, distal-side ends and proximal-side ends of the respective frame components 21A are tied and bonded to one another centering around the core wire 11.

The distal-side end of each of the frame components 21A is integrally joined to the core wire 11 by silver soldering or the like, and the respective proximal-side ends of the frame components 21A are in a state movable in a longitudinal direction to the core wire 11.

Further, on the respective outer peripheral surfaces of the tied portions where all the frame components 21A are tied, coiled bodies 13A and 13B are formed by closely winding a winding wire composed of, for example, a metallic wire material in the form of a coil. Both ends of the coiled body 13A on the proximal side and the coiled body 13B on the distal side are integrally joined to the outer peripheral surfaces of the tied portions of the frame components 21A by, for example, silver soldering.

The supporting frame 21 preferably has a thickness of at most 0.1 mm, more preferably 0.01 to 0.05 mm. In the present invention, each of the frame components 21A making up the supporting frame 21 may be made up of either a belt-like board material or a wire material.

A metal making up each of the frame components 21A is that having biocompatibility and preferably that having radiopaque property. As specific examples thereof, may be mentioned platinum, tungsten, tantalum, gold, silver, palladium, rhodium, titanium and alloys thereof, and stainless steel. Among these, tungsten or a tungsten alloy is preferably used from the reasons that it has high radiopaque property upon radiography, high strength and excellent working ability compared with other metals.

As metallic wire materials making up the coiled bodies 13A and 13B, may be used those exemplified as the metals making up the respective filter components 21A. Among these, platinum or an alloy of platinum and tungsten is particularly preferred. Such a material do not transmit X-ray, so that the leading edge position of the medical wire device can be visually observed by radiography during operation.

As already described above, in the filter unit 20, the filter 22 for capturing the free embolismic debris is provided on an inner peripheral surface of the supporting frame 21, specifically, on an inner peripheral surface of a distal-side portion in the supporting frame 21 along a circumferential surface of the spheroid formed by the supporting frame 21 in a state that the proximal-side edge of the filter 22 has been located in the vicinity of the greatest swelling portion (central position in the longitudinal direction) of the supporting frame 21.

No particular limitation is imposed on the form of the filter 22 making up the filter unit 20, and the filter may be variously shaped as necessary according to the end application intended.

The filter 22 is in the form of a mesh composed of a metal having radiopaque property. As examples of the metal having radiopaque property, may be mentioned tungsten, gold, platinum and alloys of these metals, and stainless steel. Among these, tungsten, gold, platinum and alloys of these metals, which are high in radiopaque property upon radiography compared with other metals, are preferably used, and tungsten or a tungsten alloy is more preferably used because of its high strength and excellent processing ability.

The filter 22 is made up of a metallic wire material having an element wire diameter of at most 10 µm, preferably 5 to 10 µm and has tensile break strength of at least 1,000 N/mm², preferably 1,400 to 4,500 N/mm².

The opening diameter (mesh size) of the filter 22 is, basically, a size to permit surely capturing embolismic debris without impeding a blood stream. Specifically, it is preferably, for example, 50 to 200 µm, more preferably 50 to 150 µm. The term "opening diameter of the filter 22" as used herein means a value corresponding to a maximum diameter of a circle included within an opening defined between metal wires making up the filter.

The opening rate of the filter 22 is preferably, for example, 65 to 99%, more preferably 80 to 95%.

In the above, the mechanical structure of the supporting frame 21 making up the filter unit 20 may be either a structure having, as its own property, a diametrically contracting and expanding function that it becomes a diametrically contracted state by its own bendable property in a state that external force has been applied and becomes an expanded state by being released from the external force, or a structure provided with a developing means for expanding or diametrically contracting the supporting frame 21, in, for example, a state movable (slidable) along a longitudinal direction of the core wire 11 so that the supporting frame is diametrically contracted by shifting the developing means to a direction of a proximal side and expanded by shifting the developing means to a direction of a distal side.

Such a medical wire device 10 as described above is percutaneously inserted into a blood vessel of a vital body through, for example, a proper guiding catheter and located in a distal region from a constriction lesion to be subjected to angioplasty to use it. The supporting frame 21 is contracted and the filter is in a folded state until the device reaches the intended site. After reaching the intended site, the supporting frame 21 is expanded to its original form, and the filter 22 is developed at the same time as the expansion of the supporting frame 21.

More specifically, there is used, for example, a method in which the medical wire device 10 is contained in a guiding catheter having an outer diameter smaller than an inner diameter of the intended site, a leading end of this catheter is percutaneously inserted into the blood vessel up to the intended site, and the medical wire device 10 is then pushed out of an opening in the leading end of the catheter. In the medical wire device 10, because the supporting frame 21 is made up of that having bendable property the filter unit 20 becomes a diametrically contracted state by being forcedly constrained by an inner peripheral surface of the catheter. The filter unit 20 is pushed out into the blood vessel at the intended site, whereby the supporting frame is released from constraint force by the inner peripheral surface of the catheter to restore its form to the expanded form before the contraction. The filter 22 is thereby developed in a state that its proximal-side edge substantially conforms to the inner diameter of the blood vessel.

When the supporting frame 21 has a structure that is expanded or contracted by the proper developing means, the developing means is moved to a direction of the proximal side, whereby the supporting frame 21 is diametrically contracted. In this state, the medical wire device 10 is contained in a guiding catheter and pushed out of an opening in the leading end of the catheter inserted into the blood vessel, and the developing means is moved to a direction of a distal side along the longitudinal direction in a state that the filter unit 20 has been located at the intended site, whereby the supporting frame 21 is expanded. The filter 22 is thereby developed in a state that its distal-side edge substantially conforms to the inner diameter of the blood vessel.

On the other hand, a catheter for placing a stent is percutaneously inserted into the blood vessel, and the stent is placed in the constriction lesion by a method according to the mechanical structure thereof (self-expandable stent or balloon expandable stent).

After a lumen is formed or secured by, for example, placing a stent, the medical wire device 10 is pulled back into the catheter in a state that the supporting frame 21 has been contracted, and the filter has been folded, thereby recovering it.

More specifically, in the case where the supporting frame 21 has the structure that it is expanded or contracted by its own contracting and expanding function, the filter unit 20 is pressed against a distal end of the catheter and constrained by the inner peripheral surface of the catheter, whereby the supporting frame 21 is diametrically contracted, and the filter 22 is thereby folded in a state that free embolismic debris have been captured.

In the case where the supporting frame 21 has the structure that it is expanded or contracted by the developing means, the developing means is moved to a direction of a proximal side along the longitudinal direction, whereby the supporting frame 21 is diametrically contracted, and the filter 22 is thereby folded in a state that free embolismic debris have been captured.

The angioplasty is conducted in accordance with such a method, whereby even when embolismic debris at a constriction lesion unexpectedly separate from an inner wall of the blood vessel and are caused to flow in a peripheral region by a blood stream upon placing the stent in the constriction lesion, the free embolismic debris can be captured by the filter unit 20 in the medical wire device 10.

According to such a medical wire device 10 as described above, the filter 22 making up the filter unit 20 is made up of a metal having high radiopaque property, whereby a surgeon is capable to easily confirm under radiography that the filter 22 is in a state surely developed in a lumen and brought into close contact with an inner wall surface without any clearance upon its development at a desired site.

For example, when an unexpected clearance is caused between the outer edge of the filter 22 and the inner wall of the lumen upon the development of the filter 22, accordingly, its condition can be grasped under the radiography, so that it is possible to take countermeasure such as repeated contraction and development of the filter or shifting of the position of the filter in a diametrically contracted state, and so free embolismic debris can be surely captured without leakage through the clearance.

The filter 22 making up the filter unit 20 is made up of a metal having high strength, whereby the metallic wire material making up the filter 22 can be made small in diameter or thin. As a result, the filter 22 can be made thin and made up as that having high flexibility. Accordingly, the filter can be developed or folded following the expansion or contraction of the supporting frame 21 without impeding the deformation of the supporting frame 21, so that the filter 22 can be surely developed to an expected form in a lumen such as a blood vessel.

In addition, the metallic wire material is made small in diameter or thin, whereby the opening diameter of the filter 22 can be made small while retaining the expected opening rate, so that free embolismic debris can be surely captured in a state that perfusion on a downstream side from a constriction lesion is scarcely impeded. Accordingly, the filter unit 20 can be made up as that improved in its function.

In particular, the filter 22 is made up of tungsten or an alloy composed mainly of tungsten, whereby such effects as described above can be surely achieved because tungsten or the alloy composed mainly of tungsten has high radiopaque property upon radiography, high strength and excellent processing ability by itself compared with other metals.

In addition, the supporting frame 21 making up the filter unit 20 is composed of that having a thin wall not thicker than the specified thickness, whereby the function inherent in the filter unit 20 that the free embolismic debris in the lumen are captured can be surely achieved as described above, and the filter unit 20 itself can be made up as that having high flexibility, thereby being capable of deforming the filter unit following the form of a lumen into which it should be inserted. As a result, high housing ability into the lumen and high operating ability within the lumen are achieved, and thus the filter unit can be made up as that adequately applicable to blood vessels having a small inner diameter and a complicated form like, for example, blood vessels (having an inner diameter of, for example, at most 3 mm) in a cerebral region.

The supporting frame 21 making up the filter unit 20 is in the form of a spheroid, and the filter 22 is provided on an inner peripheral surface of the supporting frame 21, whereby resistance to an inner wall of the lumen or the proper catheter can be made low upon insertion or recovery of the medical wire device 10, so that an operation can be smoothly conducted, and high operating ability can be surely achieved.

Although specific embodiments of the present invention have been described above, the present invention is not limited to the above-described embodiments, and various changes may be added to the present invention.

For example, the medical wire devices according to the present invention may be so constructed that a holding means for holding a stent composed of, for example, an expansible balloon may be provided at a proximal-side portion from the position in the core wire, at which the filter unit is arranged.

As the mechanical structure of the balloon, may be used any structure suitably used heretofore, and the constitution such as an outer diameter and an axial length may be set to proper dimensions as necessary for the end application intended.

According to the medical wire device of such construction, it is only necessary to conduct a puncture operation only once, which must be conducted at 2 positions (twice) of the skin of a patient when the device has such construction as shown in Fig. 1 for percutaneously inserting a medical wire device and for percutaneously inserting a catheter for placing a stent, so that burdens imposed on the patient can be lightened.

In the medical wire devices according to the present invention, the frame structure of the supporting frame making up the filter unit is not limited to the spheroid shown in Fig. 1, and it may be have various shapes as necessary for the end application intended.

For example, as illustrated in Fig. 3, the supporting frame may have the so-called "umbrella"-like frame structure that a plurality of frame components 31 each composed of a belt-like board material or a wire material are provided so as to extend in a longitudinal direction along a core wire 32 in a state that distal-side ends thereof have been fixed to the core wire 32 and the proximal-side ends thereof have been free, and expansion and contraction are made by a developing means 35. In Fig. 3, reference numerals 13A and 13B denote coiled bodies, and reference numeral 22 denotes a filter.

The developing means 35 is made up of, for example, a sleeve-like wire body 36 provided movably in a longitudinal direction in a state that a core wire 32 has been inserted into the interior thereof, and a plurality of wires 38 for development, which are each pivotably connected to a proximal-side end of its corresponding frame component 31 at one end thereof and pivotably connected to a supporting member 37 provided on an outer peripheral surface of the wire body at the other end.

Further, in the medical wire devices according to the present invention, the form of the filter is not limited to the membrane form provided along the peripheral surface of the frame structure (spheroid in the embodiment shown in Fig. 1), and the form may be those of various forms including that becomes a sheet form when developed.

The film may be provided in a state either tensed or relaxed to the frame structure when it is developed.

In addition, in the medical wire devices according to the present invention, the number of frame components making up the supporting frame, the dimension of the outer diameter and the axial length of the supporting frame, and other specific constitution may be suitably set to proper values according to lumens to which the medical wire devices are applied. For example, in medical wire devices for blood vessels, the greatest outer diameter of the supporting frame when expanded is 3 to 10 mm, and the length thereof is 15 to 18 mm.

The present invention will hereinafter be described by the following specific examples. However, the present invention is not limited to the following examples.

### <Production Example 1>

Following the construction shown in Fig. 1, a medical wire device (10) according to the present invention was produced. This medical wire device is referred to as "Wire Device 1". A specific construction is as follows.

### <Core wire>

A core wire (11) is composed of stainless steel and has an overall length of 2,700 mm, a proximal-side region (12a) has a length of 2,350 mm and a diameter of 0.35 mm, a tapered region (12B) has a length of 200 mm, and a distal-side region has a length of 150 mm and a diameter of 0.25 mm

### <Filter unit>

### (1) Supporting frame:

A supporting frame (21) has a spheroidal frame structure that 8 frame components (21A), which are belt-like board materials 0.05 mm x 0.1 mm in sizes composed of tungsten, are provided so as to extend in a longitudinal direction along the core wire in a state that a distal-side end of each of the frame components has been fixed to the core wire by silver soldering, and the proximal-side end thereof has been made freely movable to the core wire. The greatest outer diameter when it is expanded is 8.0 mm, and the length in a longitudinal direction is 18 mm. The supporting frame has a contracting and expanding function that it is expanded or contracted its own bendable property.

### (2) Filter:

A filter (22) is in the form of a mesh formed of a wire material composed of tungsten and having an element wire diameter of 10 µm and has an opening diameter of 150 µm and an opening rate of 88%. The tensile break strength of the wire material is 4,200 N/mm².

The filter is in a state that the proximal end edge thereof has been located at a central position of the longitudinal direction in the supporting frame (21) along a circumferential surface of the spheroid formed by the supporting frame (21).

### <Winding wire>

A winding wire is formed of a wire material composed of platinum (Pt) and having an element wire diameter of 50 µm and is closely wound in the form of a coil on an outer peripheral surface of a tied portion where the frame components are tied. Both ends of a coiled body (13B) formed on the distal side from the filter unit (20) are joined to the outer peripheral surfaces of the tied portions of the frame components (21A) by silver soldering, and a distal-side end of a coiled body (13A) formed on the proximal side from the filter unit (20) is joined to the outer peripheral surface of the tied portion of the frame components (21A) by silver soldering. The length of the coiled body (13A) on the proximal side is 30 mm, and the length of the coiled body (13B) on the distal side is 30 mm.

### <Production Example 2>

A medical wire device of the same construction as in Production Example 1 except that a platinum-tungsten alloy (Pt/W = 92/8) was used in place of tungsten as the metallic wire materials making up the supporting frame and filter in Production Example 1 was produced. This medical wire device is referred to as "Wire Device 2". The tensile break strength of the metallic wire material making up the filter in Wire Device 2 is 1,500 N/mm².

### <Production Example 3>

A medical wire device of the same construction as in Production Example 1 except that a tube for expanding a frame (developing means), which covered the core wire and extended in a longitudinal direction, was provided at a proximal-side end of the supporting frame making up the filter unit in Production Example 1 so as to contract the supporting frame by pulling this developing means on the proximal side and expand the supporting frame by pressing the developing means on the distal side, and a wire in a straight form having a diameter of 0.20 mm was used as the core wire, was produced. The tube for expanding the frame is composed of a Ti-Ni alloy and has an outer diameter of 0.36 mm, an inner diameter of 0.21 mm and a length of 2,250 mm. This medical wire device is referred to as "Wire Device 3".

### <Comparative Production Example 1>

A comparative medical wire device was produced in the same manner as in Production Example 1 except that a titanium-nickel alloy was used in place of tungsten as the metallic wire materials making up the supporting frame and filter in Production Example 1. This medical wire device is referred to as "Comparative Wire Device 1".

### <Comparative Production Example 2>

A comparative medical wire device was produced in the same manner as in Production Example 3 except that with respect to the metallic wire materials making up the supporting frame and filter in Production Example 3, the supporting frame was made up of a titanium-nickel alloy in place of tungsten, and the filter was made up of a mesh (opening rate: 50%) made of polyarylate. This medical wire device is referred to as "Comparative Wire Device 2".

With respect to the respective Wire Devices 1 to 3 and Comparative Wire Devices 1 and 2 produced in the above-described manner, a comparison test was carried out on radiopacity performance.

Under fluoroscopy with X ray, a microcatheter having an inner diameter of 0.55 mm, in which a wire device was set, was inserted up to an origin part of an internal carotid artery through a femoral artery of a rabbit, another microcatheter was inserted likewise, and a filter was developed at an arbitrary position while pouring a contrast medium as needed to observe the relationship between the filter and the inner wall of the artery. As a result, in all the Wire Devices 1 to 3 according to the present invention, it could be confirmed that the filter is developed in a state that an opening defined by a proximal-side edge thereof comes into close contact with the inner wall surface of the internal carotid artery without any clearance. It could be additionally confirmed that a good close-contact state is achieved by contracting and re-developing the filter even when the state of close contact is bad.

On the other hand, in Comparative Wire device 1 and Comparative Wire device 2, their filters could not be observed under fluoroscopy with X ray to grasp the relationship between the filter and the inner wall of the artery.

In the state that the filter had been developed, polystyrene beads having a particle diameter ranging from 200 to 500 µm were dispersed in physiological saline so as to give a proper concentration and then poured through the microcatheter from which the contrast medium was poured. As a result, with respect to all the Wire Devices 1 to 3 according to the present invention, it was confirmed that the polystyrene beads of almost 100% to the amount poured can be recovered. On the other hand, with respect to both Comparative Wire Device 1 and Comparative Wire Device 2, it was confirmed that the recovery rate was about 70%, and so these devices cannot surely capture free embolismic debris and cannot be utilized in practical use. The reason is considered to be attributable to the fact that the close contact of the side edge of the filter with the inner wall surface of the artery could not be observed, and so the polystyrene beads were leaked from a clearance caused.

With respect to the Wire Devices 1 to 3 produced in the above-described manner, such a substitute experiment as described below was carried out.

A substitute blood vessel imitated from a blood vessel (having an inner diameter of 5 mm) in a cerebral region of a vital body was produced, and a sample (substitute for blood) containing fine particles having a particle diameter ranging from 200 to 500 µm was passed through the substitute blood vessel under the same flow velocity and flow rate conditions as those of the blood vessel in the cerebral region in a state that the wire device had been placed at the appointed position in the substitute blood vessel using a proper catheter.
(1) Developed state of filter
   The developed state of the filter in the substitute blood vessel was observed. As a result, with respect to all the Wire Devices 1 to 3, it was confirmed that the filter is developed in a state that an opening defined by a proximal-side edge thereof has substantially conformed to a vertical section of the substitute blood vessel.
(2) Measurement of flow velocity
   Measurement of a flow velocity was conducted at a position on a distal side (downstream side) from the position where the medical wire was arranged. As a result, with respect to all the Wire Devices 1 to 3, it was confirmed that the degree of reduction in flow velocity is within 10% of the flow velocity set.
(3) Fine particles passed through filter
   The sample passed through the filter was collected and observed through a microscope. As a result, with respect to all the Wire Devices 1 to 3, it was confirmed that the number of fine particles passed through the filter is substantially 0.
(4) Operating ability within lumen

With respect to the operating ability upon insertion and recovery of the medical wire device, evaluation was made on the basis of feeling at the time the medical wire device was actually inserted into the substitute blood vessel. As a result, with respect to all the Wire Devices 1 to 3, it was confirmed that the wire device can be introduced into the appointed site practically without feeling a sense of resistance such as catching on the inner wall of the substitute blood vessel.

After completion of such a substitute experiment as described above, the inner wall of the substitute blood vessel was examined. As a result, it was confirmed that a damaged portion is scarcely observed.

As described above, according to the medical wire devices related to the present invention, it was confirmed that the function inherent in the filter unit that embolismic debris are captured by the filter unit can be surely achieved, high radiopaque property, high operating ability and high housing ability are attained, and the function as the medical wire device having the filter unit can be improved.

## Claims

1. A medical wire device comprising a filter unit which is to be located, upon angioplasty of a constriction lesion in a lumen, on a distal side from the constriction lesion for capturing free embolismic debris separated from an inner wall of the constriction lesion, and a wire provided with the filter unit at its distal-side end,
wherein a filter making up the filter unit is made up of a metal having radiopaque property.

2. A medical wire device comprising a filter unit which is to be located, upon angioplasty of a constriction lesion in a lumen, on a distal side from the constriction lesion for capturing free embolismic debris separated from an inner wall of the constriction lesion, and a wire provided with the filter unit at its distal-side end,
wherein a filter making up the filter unit is in the form of a mesh composed of a metallic wire material having an element wire diameter of at most 10 µm and has tensile break strength of at least 1,000 N/mm².

3. The medical wire device according to claim 1 or 2, wherein the filter unit comprises a supporting frame formed diametrically contractible and expansible and composed of a metallic wire material, and a filter provided so as to be folded by diametrically contracting the supporting frame and developed by expanding the supporting frame.

4. The medical wire device according to any one of claims 1 to 3, wherein the metallic wire material making up the filter is tungsten or a tungsten-containing alloy.

5. The medical wire device according to claim 3 or 4, wherein the supporting frame making up the filter unit is formed by providing a plurality of frame components each composed of a metallic wire material so as to extend in a longitudinal direction along a spheroidal surface, and the filter is provided along an inner peripheral surface of a distal-side portion in the supporting frame.

6. The medical wire device according to any one of claims 3 to 5, wherein the supporting frame making up the filter unit is in a state that one end of a distal-side end and a proximal-side end has been fixed to the wire, and the other end is movable along a longitudinal direction to the wire, and is so constructed that it becomes a diametrically contracted state by its own bendable property under a state that external force has been applied, and becomes an expanded state by being released from the external force.

7. The medical wire device according to any one of claims 3 to 6, wherein the supporting frame is made up of a metal having radiopaque property.

8. The medical wire device according to any one of claims 1 to 7, wherein the opening diameter of the filter making up the filter unit is 50 to 200 µm.

9. The medical wire device according to any one of claims 1 to 8, wherein the opening rate of the filter is 65 to 99%.

10. The medical wire device according to any one of claims 3 to 9, wherein the thickness of the supporting frame is 0.01 to 0.1 mm.

11. The medical wire device according to any one of claims 1 to 10, which comprises a holding means for holding an intracorporeally indwelling device for formation of a lumen at a position on a proximal side from the filter unit in the wire.
